# EUROPEAN PATENT APPLICATION

(11) **EP 3 384 935 A1**
(43) Date of publication of application: **10.10.2018**
(21) Application number: 16871097.8
(22) Date of filing: 02.12.2016
(51) Int. Cl.: A61K 47/50, A61K 38/00

(54) **PROTEIN COMPLEX USING FATTY ACID DERIVATIVE, AND PREPARATION METHOD THEREFOR**

(30) Priority: 02.12.2015 KR 20150170929
(71) Applicant: Hanmi Pharm. Co., Ltd., Hwaseong-si, Gyeonggi-do 18536 (KR)
(72) Inventor: KIM, Dae Jin, Hwaseong-si Gyeonggi-do 18469 (KR); PARK, Young Jin, Hwaseong-si Gyeonggi-do 18469 (KR); BAE, Sung Min, Hwaseong-si Gyeonggi-do 18469 (KR); JUNG, Sung Youb, Hwaseong-si Gyeonggi-do 18469 (KR); KWON, Se Chang, Hwaseong-si Gyeonggi-do 18469 (KR)
(74) Representative: Andrews, Robert
(86) International application number: PCT/KR2016/014144
(87) International publication number: WO 2017/095201

(57) **Abstract**

The present invention relates to a protein conjugate in which a physiologically active polypeptide and a biocompatible material are linked through a fatty acid derivative, thus having an extended duration of physiological activity compared to that of a natural type, and a method of preparing the same. The protein conjugate of the present invention in which a biocompatible material, fatty acid, and a physiologically active polypeptide are linked was confirmed to have an increased half-life of the physiologically active polypeptide, and thus can be widely used in the field of protein drugs.

## Description

### [Technical Field]

The present invention relates to a protein conjugate, in which a physiologically active polypeptide and a biocompatible material are linked through a fatty acid derivative, thus having an extended duration of physiological activity compared to that of the natural type, and a method of preparing the same.

### [Background Art]

Generally, physiologically active polypeptides have low stability and are thus easily denatured, degraded by proteases in the blood, and easily removed by the kidneys or livers. Accordingly, to maintain blood concentrations and titers of protein drugs containing these physiologically active polypeptides as a pharmacological component, it is necessary to frequently administer these protein drugs to patients. However, in the case of protein drugs mostly administered to patients in the form of injections, frequent administration via injections for the maintenance of the blood concentration of the physiologically active polypeptides causes severe pain to the patients. To solve these problems, many efforts have been made to maximize the efficacy of protein drugs by increasing their blood stability and maintaining their blood concentration at a high level for a long period of time. These long-acting formulations of protein drugs should not induce immune responses in patients while increasing the stability of the protein drugs.

Conventionally, as a method for stabilizing proteins and inhibiting clearance thereof in kidney and contact with proteases, a protein pegylation method where a highly soluble polymer such as polyethylene glycol (hereinafter, "PEG") is chemically added to the surface of protein drugs has been used. It has been known that PEG is effective in stabilizing proteins by non-specifically binding to a specific site or various sites of the target protein to increase the solubility thereof without causing any particular side effects and effective in preventing the hydrolysis of proteins (Sada et al., J. Fermentation Bioengineering 71: 137 to 139, 1991). Although the stability of a protein may increase due to the binding of PEG thereto, the pegylation method has problems in that the titer of the physiologically active protein may be significantly reduced, the reactivity of PEG with the protein is reduced as the molecular weight of PEG increases, and the increase of half-life of the protein is not sufficient.

Additionally, conventionally, a method of chemically adding a fatty acid to the surface of a physiologically active polypeptide drug has also been used. As such, it has been difficult to implement the effect of a long-lasting drug by linking a fatty acid to the surface thereof.

Accordingly, in the related field, there is still a need for the development of a method which can further stabilize a physiologically active polypeptide *in vivo,* inhibit clearance in kidney, and maintain the blood half-life of the physiologically active polypeptide at a high level.

### [Disclosure]

### [Technical Problem]

The present inventors have confirmed that a physiologically active polypeptide can be further stabilized and clearance in kidney can be inhibited, thereby significantly increasing the blood half-life of a physiologically active polypeptide, by linking a physiologically active polypeptide with a biocompatible material, which can improve *in vivo* stability of proteins, through a covalent bond using a fatty acid derivative as a linker, rather than increasing the stability of a protein through pegylation, thereby completing the present invention.

### [Technical Solution]

An object of the present invention is to provide a protein conjugate in which a physiologically active polypeptide and a biocompatible material are linked through a fatty acid derivative.

Another object of the present invention is to provide a method for preparing the protein conjugate.

Still another object of the present invention is to provide a protein conjugate, in which a physiologically active polypeptide and a biocompatible material are linked through a fatty acid, so as to increase the blood half-life of a physiologically active protein. Specifically, the physiologically active polypeptide and the biocompatible material may be linked through a reactive group of the fatty acid, which is a linker.

### [Advantageous Effects of the Invention]

Since it was confirmed that the protein conjugate in which a biocompatible material, a fatty acid derivative, and a physiologically active polypeptide are linked has an increased blood half-life of the physiologically active polypeptide, the protein conjugate can be widely used in the field of protein drugs.

### [Brief Description of Drawings]

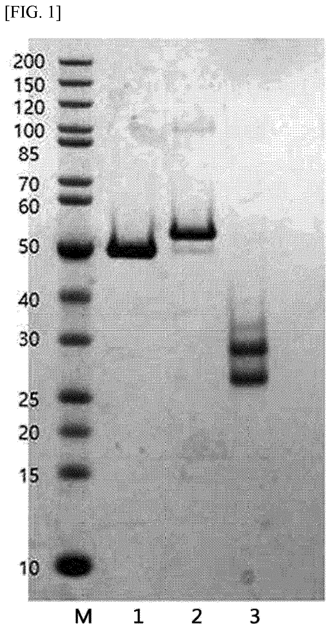

FIG. 1 shows the results of SDS-PAGE with regard to an immunoglobulin Fc conjugate; and a conjugate where a triple agonist, which can simultaneously activate GLP-1, glucagon, and GPI receptors, and an immunoglobulin Fc are linked through a fatty acid derivative, prepared according to an embodiment of the present invention (M represents a marker, lane 1 represents an unreduced immunoglobulin Fc conjugate, and lanes 2 and 3 represent an unreduced protein conjugate and a reduced protein conjugate, respectively).

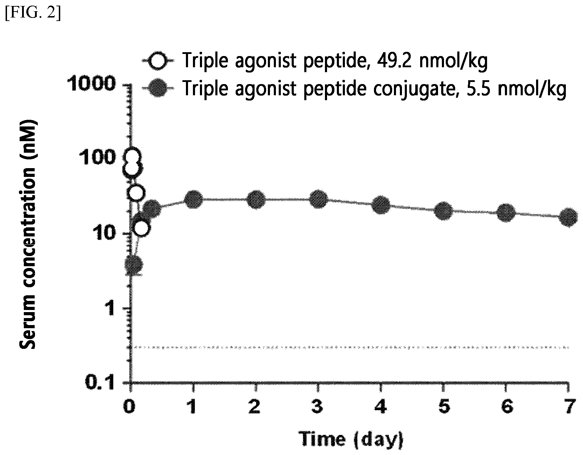

FIG. 2 shows the measurement results of *in vivo* pharmacokinetics with regard to an immunoglobulin Fc conjugate; and a conjugate where a triple agonist and an immunoglobulin Fc are linked through a fatty acid derivative, prepared according to an embodiment of the present invention. Specifically, the conventional triple agonist was used as a comparative group to show changes in the blood concentration of drugs over time.

### [Best Mode]

An aspect of the present invention provides a protein conjugate in which a physiologically active polypeptide and a biocompatible material are linked through a fatty acid derivative.

In a specific embodiment, in the protein conjugate according to the present invention, the physiologically active polypeptide and the biocompatible material may each be linked to a fatty acid derivative by a covalent bond. Specifically, the fatty acid derivative may act as a linker that connects the physiologically active polypeptide with the biocompatible material.

In another specific embodiment, the fatty acid derivative included in the protein conjugate according to the present invention may have at least two reactive groups that are linked directly or through a linker to a fatty acid backbone, and the fatty acid derivative may be linked to the physiologically active polypeptide and the biocompatible material through each of the reactive groups.

In still another specific embodiment, the reactive groups of the fatty acid derivative included in the protein conjugate according to the present invention may be each independently 2,5-dioxopyrrolidinyl, 2,5-dioxopyrrolyl, aldehyde, aryl disulfide, heteroaryl disulfide, haloacetamide, or C₇₋₁₀ alkynyl.

In still another specific embodiment, the reactive groups of the fatty acid derivative included in the protein conjugate according to the present invention may be maleimide, *N*-hydroxysuccinimide, succinimide, C₁₋₄ alkylene aldehyde, orthopyridyl disulfide (OPSS), iodoacetamide (IA), haloacetamide which comprises bromine, fluorine, chlorine, or astatin instead of iodine, difluorocyclooctyne (DIFO), dibenzocyclooctyne (DIBO), dibenzo-aza-cyclooctyne (DIBAC or DBCO), biarylazacyclooctynones (BARAC), tetramethylthiacycloheptyne (TMTH), bicyclononyne (BCN) Sondheimer diyne, cyclooctyne (OCT), monofluorinated cyclooctyne (MOFO), dimethoxyazacyclooctyne (DIMAC), 2,3,6,7-tetramethoxy-dibenzocyclooctyne (TMDIBO), sulfonylated dibenzocyclooctyne (S-DIBO), carboxymethylmonobenzocyclooctyne (COMBO), pyrrolocyclooctyne (PYRROC), or alkyne.

In still another specific embodiment, the fatty acid derivative as a linker included in the protein conjugate according to the present invention may be C₁₋₃ alkylamino or a (C₁₋₃ alkoxy)ₙ(C₁₋₃ alkylamino) chain.

In still another specific embodiment, at least one linked material in which the physiologically active polypeptide and the fatty acid derivative are linked may be conjugated to the biocompatible material included in the protein conjugate according to the present invention.

In still another specific embodiment, the physiologically active polypeptide included in the protein conjugate according to the present invention may be a hormone, cytokine, interleukin, interleukin-binding protein, enzyme, antibody, growth factor, transcription factor, blood factor, vaccine, structural protein, ligand protein or receptor, cell surface antigen, or receptor antagonist.

In still another specific embodiment, the physiologically active polypeptide included in the protein conjugate according to the present invention may be selected from the group consisting of:
incretins, which include glucagon-like peptide-1 (GLP-1), glucagon, gastric inhibitory polypeptides (GIPs), oxyntomodulin, xenin, insulin, cholecystokinin (CCK), amylin, gastrin, ghrelin, and peptide YY (PYY) that regulate blood glucose levels in the stomach or intestines and body weight;
adipokines, which include leptin, adiponectin, adipolin, apelin, and cartonectin that are secreted from adipose tissue;
neuropeptides, which include kisspeptin, nesfatin-1 that are secreted from the brain;
peptides or proteins, which include irisin, myonectin, decorin, follistatin, and musclin that are secreted from muscle; and
vasoactive intestinal peptides, natriuretic peptides, granulocyte colony-stimulating factor (G-CSF), human growth hormone (hGH), erythropoietin (EPO), growth hormone-releasing hormone, growth hormone-releasing peptides, interferons, interferon receptors, G protein-coupled receptors, interleukins, interleukin receptors, enzymes, interleukin-binding proteins, cytokine-binding proteins, macrophage-activating factors, macrophage peptides, B cell factors, T cell factors, protein A, allergy-inhibiting factors, necrosis glycoproteins, immunotoxins, lymphotoxins, tumor necrosis factors, tumor suppressors, metastasis growth factors, α-1 antitrypsin, albumin, α-lactalbumin, apolipoprotein-E, high-glycosylated erythropoietin, angiopoietins, hemoglobins, thrombin, thrombin receptor-activating peptides, thrombomodulin, blood factor VII, blood factor VIIa, blood factor VIII, blood factor IX, blood factor XIII, plasminogen activators, fibrin-binding peptides, urokinase, streptokinase, hirudin, protein C, C-reactive protein, renin inhibitors, collagenase inhibitors, superoxide dismutase, platelet-derived growth factor, epithelial growth factor, epidermal growth factor, angiostatin, angiotensin, bone morphogenetic growth factor, bone morphogenetic protein, calcitonin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, cholecystokinin, pancreatic polypeptides, gastrin-releasing peptides, corticotropin-releasing factor, thyroid-stimulating hormone, autotaxin, lactoferrin, myostatin, cell surface antigens, virus-derived vaccine antigens, monoclonal antibody, polyclonal antibody, and antibody fragments.

In still another specific embodiment, the physiologically active polypeptide according to the present invention may simultaneously activate at least two receptors.

In still another embodiment, the physiologically active polypeptide according to the present invention may be selected from derivatives of natural-type physiologically active polypeptides instead of those present in a natural type. The derivatives of physiologically active polypeptides may refer to those which have been altered in their binding affinity to native receptors or those which have been modified in their physicochemical properties, such as increased water solubility and reduced immunogenicity, through chemical modifications such as substitution, insertion, and deletion of amino acids, addition of glycans, removal of glycans, insertion of non-natural amino acids, insertion of rings, and methyl residues, and the derivatives of physiologically active polypeptides may also include artificial peptides engineered to have binding affinity for at least two different receptors.

In still another specific embodiment, the biocompatible material included in the protein conjugate according to the present invention may be selected from the group consisting of polyethylene glycol (PEG), cholesterol, albumin and a fragment thereof, an albumin-binding material, a polymer of repeating units of a particular amino acid sequence, an antibody, an antibody fragment, an FcRn-binding material, *in vivo* connective tissue or a derivative thereof, a nucleotide, fibronectin, transferrin, a saccharide, a polymer, and a combination thereof.

In still another specific embodiment, the FcRn-binding material included in the protein conjugate according to the present invention may be a polypeptide including an immunoglobulin Fc region.

In still another specific embodiment, the immunoglobulin Fc region included in the protein conjugate according to the present invention may be aglycosylated.

In still another specific embodiment, the immunoglobulin Fc region included in the protein conjugate according to the present invention may further have a hinge region.

In still another specific embodiment, the immunoglobulin Fc region included in the protein conjugate according to the present invention may be selected from the group consisting of IgG, IgA, IgD, IgE, IgM, a combination thereof, and a hybrid thereof.

In still another specific embodiment, the immunoglobulin Fc region included in the protein conjugate according to the present invention may be an IgG4 Fc fragment.

In still another specific embodiment, the fatty acid derivative included in the protein conjugate according to the present invention may be a saturated or unsaturated C₁₋₄₀ fatty acid.

In still another specific embodiment, the fatty acid included in the protein conjugate according to the present invention may be a fatty acid selected from the group consisting of formic acid (HCOOH), acetic acid (CH₃COOH), propionic acid (C₂H₅COOH), butyric acid (C₃H₇COOH), valeric acid (C₄H₉COOH), caproic acid (C₅H₁₁COOH), enanthic acid (C₆H₁₃COOH), caprylic acid (C₇H₁₅COOH), pelargonic acid (C₈H₁₇COOH), capric acid (C₉H₁₉COOH), undecylic acid (C₁₀H₂₁COOH), lauric acid (C₁₁H₂₃COOH), tridecylic acid (C₁₂H₂₅COOH), myristic acid (C₁₃H₂₇COOH), pentadecylic acid (C₁₄H₂₉COOH), palmitic acid (C₁₅H₃₁COOH), heptadecylic acid (C₁₆H₃₃COOH), stearic acid (C₁₇H₃₅COOH), nonadecanoic acid (C₁₈H₃₇COOH), arachidic acid (C₁₉H₃₉COOH), behenic acid (C₂₁H₄₃COOH), lignoceric acid (C₂₃H₄₇COOH), cerotic acid (C₂₅H₅₁COOH), heptacosanoic acid (C₂₆H₅₃COOH), montanic acid (C₂₈H₅₇COOH), melissic acid (C₂₉H₅₉COOH), lacceric acid (C₃₁H₆₃COOH), acrylic acid (CH₂=CHCOOH), crotonic acid (CH₃CH=CHCOOH), isocrotonic acid (CH₃CH=CHCOOH), undecylenic acid (CH₂=CH(CH₂)₈COOH), oleic acid (C₁₇H₃₃COOH), elaidic acid (C₁₇H₃₃COOH), cetoleic acid (C₂₁H₄₁COOH), erucic acid (C₂₁H₄₁COOH), brassidic acid (C₂₁H₄₁COOH), sorbic acid (C₅H₇COOH, F2), linoleic acid (C₁₇H₃₁COOH, F2), linolenic acid (C₁₇H₂₉COOH, F3), arachidonic acid (C₁₉H₃₁COOH, F4), propiolic acid (CH=CCOOH), and stearolic acid (C₁₇H₃₁COOH, F1), or a derivative thereof.

In still another specific embodiment, the fatty acid included in the protein conjugate according to the present invention may be a fatty acid of palmitic acid, myristic acid, stearic acid, or oleic acid, or a derivative thereof.

Another aspect of the present invention provides a method for preparing a protein conjugate, which includes (a) linking a physiologically active peptide and a biocompatible material through a fatty acid derivative having at least two reactive groups; and (b) separating the protein conjugate, which is a reaction product of step (a).

In a specific embodiment, in the method according to the present invention, step (a) may include
(a1) linking any one of the biocompatible material and the physiologically active polypeptide to one reactive group of the fatty acid derivative;
(a2) separating the linked material, in which any one of the biocompatible material and the physiologically active polypeptide is linked to the fatty acid derivative, from the reaction mixture of step (a1); and
(a3) linking the other of the biocompatible material and the physiologically active polypeptide to another reactive group of the fatty acid derivative in the linked material separated in step (a2), and producing a protein conjugate in which the reactive groups of the fatty acid are linked to each of the physiologically active polypeptide and the biocompatible material.

In another specific embodiment, in the method according to the present invention, step (a1) and step (a3) may be performed in the presence of a reducing agent.

In still another specific embodiment, in the method according to the present invention, the reducing agent may be sodium cyanoborohydride (NaCNBH₃), sodium borohydride, dimethylamine borane, a picoline borane complex, or borane pyridine.

In still another specific embodiment, in step (a1) of the method according to the present invention, the reaction molar ratio of the physiologically active polypeptide to the fatty acid derivative may be in the range of 1:1 to 1:20 and the reaction molar ratio of the biocompatible material to the fatty acid derivative may be in the range of 1:1 to 1:20.

In still another specific embodiment, in step (a3) of the method according to the present invention, the reaction molar ratio of the linked material separated in step (a2) to the biocompatible material or the physiologically active polypeptide may be in the range of 1:0.5 to 1:20.

### [Mode of the Invention]

An aspect of the present invention provides a protein conjugate in which a physiologically active polypeptide and a biocompatible material are linked through a fatty acid derivative. In particular, in the protein conjugate of the present invention, the physiologically active polypeptide and the biocompatible material may each be linked to the fatty acid derivative by a covalent bond.

As used herein, the term "physiologically active polypeptide", which may be a constituent constituting a moiety of the conjugate, is a general term for a polypeptide having a certain physiological action *in vivo,* and physiologically active polypeptides have a common characteristic of a polypeptide structure and have various physiological activities. The physiologically active polypeptide may also include which has the role of correcting abnormal pathological conditions caused by deficiency or excessive secretion of materials, which are involved in regulation of functions *in vivo,* by adjusting genetic expression and physiological functions, and may also include general protein therapeutic agents. Additionally, the term "physiologically active polypeptide" is a concept including not only natural polypeptides but also all of the derivatives thereof.

With regard to the conjugate of the present invention, the type and size of the physiologically active polypeptide are not particularly limited as long as the physiologically active polypeptide can exhibit the increase of blood half-life through the conjugate structure of the present invention.

The physiologically active polypeptide of the present invention may be a hormone, cytokine, interleukin, interleukin-binding protein, enzyme, antibody, growth factor, transcription factors, blood factor, vaccine, structural protein, ligand protein or receptor, cell surface antigen, or receptor antagonist.

In another specific embodiment, the physiologically active polypeptide included in the protein conjugate according to the present invention may be selected from the group consisting of:
incretins, which include glucagon-like peptide-1 (GLP-1), glucagon, gastric inhibitory polypeptides (GIPs), oxyntomodulin, xenin, insulin, cholecystokinin (CCK), amylin, gastrin, ghrelin, and peptide YY (PYY) that regulate blood glucose levels in the stomach or intestines and body weight;
adipokines, which include leptin, adiponectin, adipolin, apelin, and cartonectin that are secreted from adipose tissue;
neuropeptides, which include kisspeptin, nesfatin-1 that are secreted from the brain;
peptides or proteins, which include irisin, myonectin, decorin, follistatin, and musclin that are secreted from muscle; and
vasoactive intestinal peptides, natriuretic peptides, granulocyte colony-stimulating factor (G-CSF), human growth hormone (hGH), erythropoietin (EPO), growth hormone-releasing hormone, growth hormone-releasing peptides, interferons, interferon receptors, G protein-coupled receptors, interleukins, interleukin receptors, enzymes, interleukin-binding proteins, cytokine-binding proteins, macrophage-activating factors, macrophage peptides, B cell factors, T cell factors, protein A, allergy-inhibiting factors, necrosis glycoproteins, immunotoxins, lymphotoxins, tumor necrosis factors, tumor suppressors, metastasis growth factors, α-1 antitrypsin, albumin, α-lactalbumin, apolipoprotein-E, high-glycosylated erythropoietin, angiopoietins, hemoglobins, thrombin, thrombin receptor-activating peptides, thrombomodulin, blood factor VII, blood factor VIIa, blood factor VIII, blood factor IX, blood factor XIII, plasminogen activators, fibrin-binding peptides, urokinase, streptokinase, hirudin, protein C, C-reactive protein, renin inhibitors, collagenase inhibitors, superoxide dismutase, platelet-derived growth factor, epithelial growth factor, epidermal growth factor, angiostatin, angiotensin, bone morphogenetic growth factor, bone morphogenetic protein, calcitonin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, cholecystokinin, pancreatic polypeptides, gastrin-releasing peptides, corticotropin-releasing factor, thyroid-stimulating hormone, autotaxin, lactoferrin, myostatin, cell surface antigens, virus-derived vaccine antigens, monoclonal antibody, polyclonal antibody, and antibody fragments.

In still another embodiment, the physiologically active polypeptide according to the present invention may be selected from derivatives of natural-type physiologically active polypeptides instead of those present in a natural type. The derivatives of physiologically active polypeptides may refer to those which have been altered in their binding affinity to native receptors or those which have been modified in their physicochemical properties such as increased water solubility and reduced immunogenicity, through chemical modifications such as substitution, insertion, and deletion of amino acids, addition of glycans, removal of glycans, insertion of non-natural amino acids, insertion of rings, and methyl residues, and the derivatives of physiologically active polypeptides may also include artificial peptides engineered to have binding affinity for at least two different receptors.

As used herein, the terms such as "physiologically active polypeptide", "physiologically active protein", "active protein", and "protein drug" represent a polypeptide or protein that is antagonistic to physiological phenomena *in vivo,* and these terms can be interchangeably used with each other.

As used herein, the term "biocompatible material", which may be a constituent constituting a moiety of the conjugate, refers to a material that can bind to a physiologically active polypeptide and thereby increase its *in vivo* half-life. As used herein, the term "biocompatible material" is a material which can extend the *in vivo* half-life, and may be expressed as a "carrier", and these two terms can be used interchangeably with each other. The biocompatible material or carrier includes all of the materials that can bind to a physiologically active polypeptide and extend its half-life. For example, the biocompatible material or carrier may be selected from the group consisting of polyethylene glycol (PEG), cholesterol, albumin and a fragment thereof, an albumin-binding material, a polymer of repeating units of a particular amino acid sequence, an antibody, an antibody fragment, an FcRn-binding material, *in vivo* connective tissue or a derivative thereof, a nucleotide, fibronectin, transferrin, a saccharide, a polymer, and a combination thereof, but the biocompatible material or carrier is not limited thereto. The FcRn-binding material may be a polypeptide including an immunoglobulin Fc region, for example, an IgG Fc. The biocompatible material or carrier can bind to a physiologically active polypeptide through a fatty acid derivative.

When polyethylene glycol is used as a carrier, Recode, the technology of Ambrex, enabling the attachment of polyethylene glycol in a position-specific manner may be included, and glycopegylation, the technology of Neose Technologies, Inc. may be included. Additionally, releasable PEG technology in which polyethylene glycol is slowly removed *in vivo,* but the technologies to be included are not limited thereto, and technologies for increasing bioavailability using PEG may be included. Additionally, polymers such as polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer, a lipid polymer, chitins, and hyaluronic acid can also be bound to the conjugate of the present invention by the above technologies.

In the present invention, when albumin is used as a carrier, the protein conjugate of the present invention may be a conjugate in which albumin or a fragment thereof is covalently bonded directly to a fatty acid derivative. Additionally, the protein conjugate of the present invention may be a conjugate in which an albumin-binding material (*e.g*., an albumin-specific binding antibody or an antibody fragment thereof) is linked to a fatty acid derivative so as to be linked to albumin, although albumin itself may not be directly linked thereto. Additionally, the protein conjugate of the present invention may be a conjugate in which the linkage is formed by linking a particular peptide/protein/compound, *etc.* having a binding affinity for albumin to a fatty acid derivative, or a protein conjugate in which a fatty acid having a binding affinity for albumin itself is linked, but the protein conjugates are not limited thereto.

In the present invention, an antibody or a fragment thereof may be used as a carrier, and this may be an antibody having an FcRn-binding region or an antibody fragment thereof, or an antibody fragment not containing an FcRn-binding region (*e.g.,* Fab, *etc*.), but the antibody or a fragment thereof is not limited thereto. Specifically, in the present invention, an immunoglobulin Fc region may be used as a carrier.

An immunoglobulin Fc region is a biodegradable polypeptide metabolized *in vivo,* and thus, it is safe for use as a drug carrier. Additionally, the immunoglobulin Fc region has a lower molecular weight relative to the entire immunoglobulin molecule, and thus it has advantages in preparation, purification, and yield of a conjugate. Furthermore, due to the removal of Fab parts with high heterogeneity because of the variations in amino acid sequences among antibodies, the effects that the homogeneity of materials can be significantly increased and the possibility of inducing antigenicity in the blood can be lowered are also expected.

As used herein, the term "immunoglobulin Fc region" refers to a heavy chain constant region of immunoglobulin excluding the variable regions of the heavy chain and light chain, the heavy chain constant region 1 (CHI) and the light-chain constant region 1 (CL1) of the immunoglobulin. However, the Fc fragment may include a hinge region in the heavy chain constant region.

Additionally, the immunoglobulin Fc region of the present invention may be an extended Fc region including a part or the entirety of the heavy chain constant region 1 (CHI) and/or the light chain constant region 1 (CL1), excluding the heavy chain and the light chain variable regions of the immunoglobulin.

Such an immunoglobulin Fc region is a biodegradable polypeptide metabolized *in vivo,* and thus, it is safe for use as a drug carrier. Additionally, the immunoglobulin Fc region has a lower molecular weight relative to the entire immunoglobulin molecule, and thus it has advantages in preparation, purification, and yield of a conjugate. Furthermore, due to the removal of Fab parts with high heterogeneity because of the variations in amino acid sequences among antibodies, the effects that the homogeneity of materials can be significantly increased and the possibility of inducing antigenicity in the blood can be lowered are also expected.

In the present invention, the immunoglobulin Fc region not only includes a natural-type amino acid sequence but also a sequence variant thereof. An amino acid sequence variant(mutant) refers to an amino acid sequence in natural amino acid sequence which has a difference in at least one amino acid residue due to deletion, insertion, non-conservative or conservative substitution, or a combination thereof. For example, in an IgG Fc, the amino acid residues at positions 214 to 238, 297 to 299, 318 to 322, or 327 to 331, which are known to be important in the conjugation, may be used as suitable sites for modification.

Additionally, various other kinds of variants are possible, including one that has a deletion of a region capable of forming a disulfide bond, or a deletion of some amino acid residues at the N-terminus of native Fc or an addition of a methionine residue at the N-terminus of native Fc. Further, to remove effector functions, deletion may occur in a complement-binding site (*e.g.,* a C1q-binding site and an antibody dependent cell mediated cytotoxicity (ADCC) site). Techniques of preparing such sequence derivatives of the immunoglobulin Fc region are disclosed in International Patent Publication Nos. WO 97/34631, WO 96/32478, *etc.*

Amino acid exchanges in proteins and peptides which do not generally alter the activity of the molecules are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

In some cases, the immunoglobulin Fc region may be modified by phosphorylation, sulfation, acrylation, glycosylation, methylation, farnesylation, acetylation, amidation, *etc.*

The above-described Fc variants show biological activity identical to that of the immunoglobulin Fc fragment of present invention but have improved structural stability against heat, pH, *etc.*

Additionally, the immunoglobulin Fc region may be obtained from native forms isolated *in vivo* from humans and animals such as cows, goats, pigs, mice, rabbits, hamsters, rats, guinea pigs, *etc.,* or may be recombinants or derivatives thereof, obtained from transformed animal cells or microorganisms. Herein, the immunoglobulin Fc region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. These fragments can be isolated using size exclusion chromatography, *etc.*

Preferably, the immunoglobulin Fc region is a recombinant immunoglobulin Fc region obtained from a microorganism with regard to a human-derived Fc region.

Additionally, the immunoglobulin Fc region may be in the form of native glycan, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. The immunoglobulin Fc region obtained by removal of glycans from the Fc region shows a significant decrease in binding affinity to the complement (C1q part) and a decrease or removal of antibody-dependent cytotoxicity or complement-dependent cytotoxicity, and thus it does not induce unnecessary immune responses *in vivo.* In this regard, an immunoglobulin Fc region in a deglycosylated or aglycosylated immunoglobulin Fc region may be a more suitable form as a drug carrier to meet the original object of the present invention.

As used herein, the term "deglycosylated Fc region" refers to an Fc region in which sugar moieties are enzymatically removed, and the term "aglycosylated Fc region" refers to an Fc region which is not glycosylated and produced in prokaryotes, preferably, *E. coli.*

Meanwhile, the immunoglobulin Fc region may be derived from humans or animals including cows, goats, pigs, mice, rabbits, hamsters, rats, and guinea pigs, and preferably, it is derived from humans. In addition, the immunoglobulin (Ig) Fc region may be derived from IgG, IgA, IgD, IgE, IgM, or a combination or hybrid thereof. Preferably, it is derived from IgG or IgM, which are among the most abundant proteins in human blood, and most preferably, it is derived from IgG, which is known to enhance the half-life of ligand-binding proteins.

Meanwhile, as used herein, the term "combination" means that polypeptides encoding single-chain immunoglobulin Fc regions of the same origin are linked to a single-chain polypeptide of a different origin in the formation of a dimer or multimer. That is, a dimer or multimer may be prepared from two or more fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain immunoglobulin Fc fragment. In the present invention, various hybrid forms are possible. That is, the hybrid domain may be composed of one to four domains selected from the group consisting of CH1, CH2, CH3, and CH4 of IgG Fc, IgM Fc, IgA Fc, IgE Fc, and IgD Fc, and may include a hinge region.

Meanwhile, IgG may also be divided into the IgG1, IgG2, IgG3, and IgG4 subclasses, and the present invention may include combinations or hybrids thereof. Preferably, IgG are the IgG2 and IgG4 subclasses, and most specifically, the Fc region of IgG4 rarely having effector functions such as complement dependent cytotoxicity (CDC). In particular, the immunoglobulin Fc region for use as a carrier included in the protein conjugate of the present invention may be an aglycosylated Fc fragment derived from human IgG4. The human-derived Fc fragment can exhibit an excellent effect compared to the non-human derived Fc fragment, which acts as an antigen *in vivo* in a living human body, thereby causing undesirable immune responses such as production of new antibodies thereto.

In the present invention, a fragment of a peptide or protein may be used as a carrier for the increase of *in vivo* half-life. The fragment of a peptide or protein to be used may be an elastin-like polypeptide (ELP) consisting of a polymer of repeating units of a combination of particular amino acid sequences, and may include transferrin, which is known to have high *in vivo* stability, fibronectin, which is a constituting component of connective tissue, and derivatives thereof, but the fragments are not limited thereto, and any peptide or protein that increases *in vivo* half-life is included in the scope of the present invention.

As used herein, the term "fatty acid" may be a constituent constituting a moiety of the conjugate, and refers to a hydrocarbon chain having one carboxy group (-COOH), and monovalent carboxylic acids having the formula R-COOH are collectively referred to as fatty acids. The fatty acid of the present invention may be a saturated fatty acid or an unsaturated fatty acid depending on the bond between the carbon backbones that form the hydrocarbon chains. The unsaturated fatty acid refers to a fatty acid having at least one double bond in the bonds of the carbon backbone forming the hydrocarbon chain, and the fatty acid in which all of the bonds of the carbon backbone are single bonds is a saturated fatty acid.

Additionally, the fatty acid of the present invention may be a fatty acid having a normal chain structure or a fatty acid having a side chain in an alkyl group. Fatty acids can be classified as short-chain/medium-chain/long-chain fatty acids depending on the carbon number of a given hydrocarbon chain. In general, fatty acids can be classified as short-chain fatty acids having 1 to 6 carbon atoms, medium-chain fatty acids having 6 to 12 carbon atoms, and long-chain fatty acids having 14 or more carbon atoms. Additionally, in the case of unsaturated fatty acids, the characteristics may vary depending on the position of the double bond.

Furthermore, as used herein, the term "fatty acid derivative" may refer to a material in which two or more reactive groups are attached directly or through a linker to the fatty acid backbone described above. For example, examples of the reactive group may include 2,5-dioxopyrrolidinyl, 2,5-dioxopyrrolyl, aldehyde, aryl disulfide, heteroaryl disulfide, haloacetamide, and C₇₋₁₀ alkynyl. Specifically, the reactive group may be maleimide, *N*-hydroxysuccinimide, succinimide, formaldehyde, acetaldehyde, propionaldehyde, butyraldehyde, orthopyridyl disulfide (OPSS), iodoacetamide, haloacetamide which comprises bromine, fluorine, chlorine, or astatin instead of iodine, difluorocyclooctyne (DIFO), dibenzocyclooctyne (DIBO), dibenzo-aza-cyclooctyne (DIBAC or DBCO), biarylazacyclooctynones (BARAC), tetramethylthiacycloheptyne (TMTH), bicyclononyne (BCN) Sondheimer diyne, cyclooctyne (OCT), monofluorinated cyclooctyne (MOFO), dimethoxyazacyclooctyne (DIMAC), 2,3,6,7-tetramethoxy-dibenzocyclooctyne (TMDIBO), sulfonylated dibenzocyclooctyne (S-DIBO), carboxymethylmonobenzocyclooctyne (COMBO), pyrrolocyclooctyne (PYRROC), or alkyne, but the reactive group is not limited thereto.

Additionally, the reactive group may be linked to a fatty acid backbone through a linker including C₁₋₃ alkylamino and a (C₁₋₃ alkoxy)ₙ(C₁₋₃ alkylamino) chain (in which n is an integer of 1 to 3), but the kind of the linker is not limited thereto.

The fatty acid of the present invention may be a carboxylic acid having the formula of R-COOH, wherein the R group may include a linear or branched saturated hydrocarbon group, a saturated fatty acid or unsaturated fatty acid, a short-chain fatty acid, a medium-chain fatty acid, and a long-chain fatty acid. Specifically, the fatty acid of the present invention may be a fatty acid having 1 to 40 carbon atoms, and more specifically 4 to 30 carbon atoms that constitutes a hydrocarbon, but the fatty acid of the present invention is not limited thereto. For example, the fatty acid of the present invention may be one selected from the group consisting of formic acid (HCOOH), acetic acid (CH₃COOH), propionic acid (C₂H₅COOH), butyric acid (C₃H₇COOH), valeric acid (C₄H₉COOH), caproic acid (C₅H₁₁COOH), enanthic acid (C₆H₁₃COOH), caprylic acid (C₇H₁₅COOH), pelargonic acid (C₈H₁₇COOH), capric acid (C₉H₁₉COOH), undecylic acid (C₁₀H₂₁COOH), lauric acid (C₁₁H₂₃COOH), tridecylic acid (C₁₂H₂₅COOH), myristic acid (C₁₃H₂₇COOH), pentadecylic acid (C₁₄H₂₉COOH), palmitic acid (C₁₅H₃₁COOH), heptadecylic acid (C₁₆H₃₃COOH), stearic acid (C₁₇H₃₅COOH), nonadecanoic acid (C₁₈H₃₇COOH), arachidic acid (C₁₉H₃₉COOH), behenic acid (C₂₁H₄₃COOH), lignoceric acid (C₂₃H₄₇COOH), cerotic acid (C₂₅H₅₁COOH), heptacosanoic acid (C₂₆H₅₃COOH), montanic acid (C₂₈H₅₇COOH), melissic acid (C₂₉H₅₉COOH), lacceric acid (C₃₁H₆₃COOH), acrylic acid (CH₂=CHCOOH), crotonic acid (CH₃CH=CHCOOH), isocrotonic acid (CH₃CH=CHCOOH), undecylenic acid (CH₂=CH(CH₂)₈COOH), oleic acid (C₁₇H₃₃COOH), elaidic acid (C₁₇H₃₃COOH), cetoleic acid (C₂₁H₄₁COOH), erucic acid (C₂₁H₄₁COOH), brassidic acid (C₂₁H₄₁COOH), sorbic acid (C₅H₇COOH, F2), linoleic acid (C₁₇H₃₁COOH, F2), linolenic acid (C₁₇H₂₉COOH, F3), arachidonic acid (C₁₉H₃₁COOH, F4), propiolic acid (CH=CCOOH), and stearolic acid (C₁₇H₃₁COOH, F1), or a derivative thereof, and more specifically, palmitic acid, myristic acid, stearic acid, or oleic acid, but the fatty acid of the present invention is not limited thereto.

Additionally, the fatty acid of the present invention may be a derivative, analogue, *etc.* of the fatty acids explained above, and in particular, may be a variant in which the fatty acid-constituting hydrocarbon includes a cyclic group in addition to a linear or branched group. The cyclic group which can be contained in the hydrocarbon may be a saturated homocycle, heterocycle, aromatic condensed or non-condensed homocycle or heterocycle, and may gave an ether bond, an unsaturated bond, and a substituent.

Additionally, fatty acid derivates described in U.S. Pat. No. 8,1293,43, International Patent Publication Nos. WO 2015/067715, WO 2015/055801, WO 2013/041678, WO 2014/133324, WO 2014/009316, and WO 2015/052088 can be used as the fatty acid of the present invention, but the fatty acid of the present invention is not limited thereto. For example, the fatty acid of the present invention may include, without limitation, a multi-fatty acid including two or more carboxyl groups, materials which include a carboxylic acid (bio)isostere, phosphoric acid, or sulfonic acid instead of a carboxyl group of a fatty acid, fatty acid ester, *etc.*

In addition, with regard to the fatty acid of the present invention, derivatives and analogs of the above fatty acids already known in the art, and derivatives and analogs which can readily be produced in the state of the art are also within the scope of the present invention. The molecular weight of the fatty acid of the present invention may be in the range of 0.1 kDa to 100 kDa, and specifically 0.1 kDa to 30 kDa. As the fatty acid of the present invention, not only a single kind of a polymer but a combination of different kinds of polymers may be used, but the fatty acid of the present invention is not limited thereto.

The two or more reactive groups of a fatty acid derivative included in the protein conjugate of the present invention may be the same as or different from each other. For example, the fatty acid derivative may have a maleimide group at one reactive group and an alkyl aldehyde groups such as an aldehyde group, a propionaldehyde group, or a butyraldehyde group at the another reactive group. In a case where a fatty acid derivative which has a hydroxyl group at two reactive groups, the conjugate of the present invention can be prepared by activating the hydroxyl groups into various reactive groups described above via known chemical methods or using commercially available fatty acids having a modified reactive group(s).

The protein conjugate of the present invention may be one in which a fatty acid is linked to an amino acid residue positioned in the middle, not the N-terminus or C-terminus, of the physiologically active polypeptide or the ends of a polypeptide while a biocompatible material is linked to a part of the fatty acid which is linked to the physiologically active polypeptide. The N-terminus or C-terminus includes a region consisting of 1 to 25 amino acids at the N-terminus or C-terminus rather than the terminus itself.

The protein conjugate of the present invention may include at least one of the [physiologically active polypeptide-fatty acid derivative-biocompatible material] structure, and the elements constituting the same may be connected in a linear or branched type by a covalent bond, and the protein conjugate of the present invention may include at least one of each of the elements. Since the fatty acid of the present invention includes at least two reactive groups, the fatty acid can be covalently linked to a physiologically active polypeptide and a biocompatible material through each of the reactive groups. Additionally, at least one conjugate in which a physiologically active polypeptide and a fatty acid derivative are linked is conjugated to one biocompatible material by a covalent bond thereby forming a monomer, dimer, or multimer of the physiologically active polypeptide using the biocompatible material as a mediator, and through the same, the increase of *in vivo* activity and stability of the physiologically active polypeptide can be more effectively achieved.

In the protein conjugate of the present invention, the physiologically active polypeptide and the biocompatible material may be combined at various molar ratios.

Still another aspect of the present invention provides a method for preparing a protein conjugate, which includes (a) linking a physiologically active peptide and a biocompatible material through a fatty acid derivative; and (b) separating the protein conjugate, which is a reaction product of step (a).

The physiologically active peptide, biocompatible material, and fatty acid derivative are the same as explained above.

In step (a), the three components may be linked by covalent bonds, and the covalent bonds may occur sequentially or simultaneously. For example, in a case where a physiologically active polypeptide and a biocompatible material are each linked to the reactive groups of a fatty acid derivative having at least two reactive groups, it is advantageous to proceed with the reactions sequentially by first linking any one of the physiologically active polypeptide and the biocompatible material to one of the reactive groups of the fatty acid derivative, and then linking the other of the physiologically active polypeptide and the biocompatible material to the another reactive group of the fatty acid derivative, so as to minimize the generation of byproducts other than the targeted protein conjugate.

Accordingly, step (a) may include:
(a1) linking any one of the biocompatible material and the physiologically active polypeptide to one reactive group of the fatty acid derivative;
(a2) separating the linked material, in which any one of the biocompatible material and the physiologically active polypeptide is linked to the fatty acid derivative, from the reaction mixture of step (a1); and
(a3) linking the other of the biocompatible material and the physiologically active polypeptide to another reactive group of the fatty acid derivative in the linked material separated in step (a2), and producing a protein conjugate in which the reactive groups of the fatty acid are linked to each of the physiologically active polypeptide and the biocompatible material,
but the method is not limited thereto.

The reactions of step (a1) and step (a3) may be performed in the presence of a reducing agent, as necessary, considering the type of reactive groups of a fatty acid derivative participating in the reactions. Specific examples of the reducing agent may include sodium cyanoborohydride (NaCNBH₃), sodium borohydride, dimethylamine borane, a picoline borane complex, borane pyridine, *etc.*

In the above, steps (a2) and (b) may be performed by selecting, as necessary, appropriate methods among the conventional methods used for protein separation, considering the characteristics such as required purity and the molecular weight and charge of the resulting products. For example, various known methods, including size exclusion chromatography or ion exchange chromatography, may be applied, and as needed, a plurality of different methods may be used in combination to purify at a higher purity.

In step (a1), the reaction molar ratio of the physiologically active polypeptide to the fatty acid derivative, and the reaction molar ratio of the biocompatible material to the fatty acid derivative may each be selected in the range of 1:1 to 1:20. Specifically, the reaction molar ratio of the physiologically active polypeptide to the fatty acid derivative may be in the range of 1:2 to 1:10, and the reaction molar ratio of the biocompatible material to the fatty acid derivative may be in the range of 1:4 to 1:20. Meanwhile, in step (a3), the reaction molar ratio of the linked material separated in step (a2) to the biocompatible material or the physiologically active polypeptide may be in the range of 1:0.5 to 1:20, but the reaction molar ratio is not limited thereto.

Still another aspect of the present invention provides a protein conjugate prepared by a method for preparing the protein conjugate of the present invention, and a pharmaceutical composition containing the prepared protein conjugate. The pharmaceutical composition of the present invention may be a long-acting preparation having increased duration and stability *in vivo* compared to a natural-type physiologically active polypeptide.

The pharmaceutical composition containing the conjugate of the present invention may include a pharmaceutically acceptable carrier. For oral administration, the pharmaceutically acceptable carrier may contain a binder, a lubricant, a disintegrator, an excipient, a solubilizer, a dispersant, a stabilizer, a suspending agent, a coloring agent, a flavoring agent, *etc.* For injectable preparations, the pharmaceutically acceptable carrier may contain a buffering agent, a preservative, an analgesic, a solubilizer, an isotonic agent, a stabilizer, *etc.* For preparations for topical administration, the pharmaceutically acceptable carrier may contain a base, an excipient, a lubricant, a preservative, *etc.* The pharmaceutical composition of the present invention may be formulated into various dosage forms in combination with the above-mentioned pharmaceutically acceptable carriers. For example, for oral administration, the pharmaceutical composition may be formulated into tablets, troches, capsules, elixirs, suspensions, syrups, wafers, *etc.* For injectable preparations, the pharmaceutical composition may be formulated into a single-dose ampoule or multidose form. The pharmaceutical composition may also be formulated into solutions, suspensions, tablets, pills, capsules, sustained-release preparations, *etc.*

Meanwhile, examples of carriers, excipients, and diluents suitable for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.*

Additionally, the pharmaceutical composition of the present invention may further contain a filler, an anti-coagulant, a lubricant, a humectant, a flavoring agent, a preserving agent, *etc.*

Hereinafter, the present invention will be described in more detail with reference to the following examples. However, the following examples are for illustrative purposes only and are not intended to limit the scope of the present invention.

### Preparation Example 1: Synthesis of intermediate (1), 2-(2-(2-aminoethoxy)ethoxy)-N-(2-(2-(2,2-dimethoxyethoxy)ethoxy)ethyl)acetamide, for preparation of fatty acid derivatives having at least two reactive groups

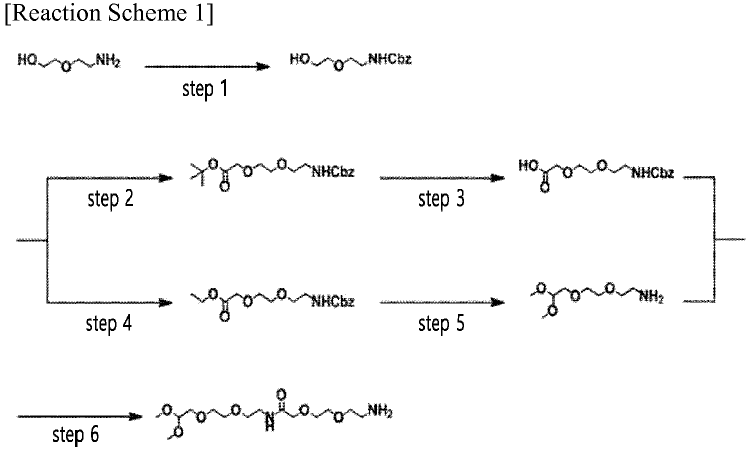

### Step 1. Preparation of benzyl 2-(2-hydroxyethoxy)ethylcarbamate

After dissolving 2-(2-aminoethoxy)ethanol (150 mL, 1.459 mol) in tetrahydrofuran (THF, 5 L), triethylamine (229 mL, 1.645 mol) and benzyl chloroformate (211 mL, 1.495 mol) were added thereto and the mixture was stirred for 12 hours. The solid was filtered off, washed with ethyl acetate, and the filtrate was concentrated. The concentrate was purified by column chromatography to give the title compound (202 g).

¹H NMR (300 MHz, CDCl₃) δ 7.37-7.29 (m, 5H), 5.25 (br, 1H), 5.10 (s, 2H), 4.13-4.11 (m, 2H), 3.57-3.54 (m, 4H), 3.43-3.38 (m, 2H), 2.24 (br, 1H).

### Step 2. Preparation of t-butyl 3-oxo-1-phenyl-2,7,10-trioxa-4-azadodecan-12-oate

After dissolving the benzyl 2-(2-hydroxyethoxy)ethylcarbamate (129 g, 0.539 mol) obtained in Step 1 in THF (2 L), potassium t-butoxide (60.5 g, 0.593 mol) was added dropwise thereto at 0°C. After 30 minutes, t-butyl bromoacetate was added thereto and the mixture was stirred at 0°C for 3 hours and further stirred at room temperature for 15 hours. The reaction was stopped by adding water to the reaction solution, and the resultant was concentrated and extracted with ethyl acetate. The organic layer was dried over anhydrous magnesium sulfate and filtered. The filtrate was concentrated and purified by column chromatography to give the title compound (87.5 g).

¹H NMR (300 MHz, CDCl₃) δ 7.36-7.30 (m, 5H), 5.38 (br, 1H), 5.10 (s, 2H), 4.00 (s, 2H), 3.70-3.64 (m, 4H), 3.60-3.56 (m, 2H), 3.43-3.40 (m, 2H), 1.46 (s, 9H).

### Step 3. Preparation of 3-oxo-1-phenyl-2,7,10-trioxa-4-azadodecan-12-oic acid

After dissolving the t-butyl 3-oxo-1-phenyl-2,7,10-trioxa-4-azadodecan-12-oate (22.0 g, 62.249 mol) obtained in Step 2 in dichloromethane (138 mL), trifluoroacetic acid (138 mL) was added thereto and the mixture was stirred at room temperature for 5 hours. The reaction solution was filtered and concentrated to give the title compound (13.0 g).

¹H NMR (300 MHz, CDCl₃) δ 7.36-7.31 (m, 5H), 5.11 (s, 2H), 4.15 (s, 2H), 3.74-3.58 (m, 6H), 3.42-3.40 (m, 2H).

### Step 4. Preparation of ethyl 3-oxo-1-phenyl-2,7,10-trioxa-4-azadodecan-12-oate

After dissolving the benzyl 2-(2-hydroxyethoxy)ethylcarbamate (15.0 g, 62.691 mmol) obtained in Step 1 in THF (240 mL), potassium *t*-butoxide (7.0 g, 62.691 mmol) was added dropwise thereto at 0°C. After 30 minutes, ethyl bromoacetate was added thereto and the mixture was stirred at 0°C for 3 hours and further stirred at room temperature for 15 hours. The reaction was stopped by adding water to the reaction solution, and the resultant was concentrated and extracted with ethyl acetate. The organic layer was dried over anhydrous magnesium sulfate and filtered. The filtrate was concentrated and purified by column chromatography to give the title compound (8.6 g).

¹H NMR (300 MHz, CDCl₃) δ 7.36-7.30 (m, 5H), 5.32 (br, 1H), 5.10 (s, 2H), 4.19 (q, 2H), 4.12 (s, 2H), 3.72-3.65 (m, 4H), 3.59-3.56 (m, 2H), 3.41-3.38 (m, 2H), 1.26 (t, 3H).

### Step 5. Preparation of 2-(2-(2,2-dimethoxyethoxy)ethoxy)ethan-1-amine

After dissolving the ethyl 3-oxo-1-phenyl-2,7,10-trioxa-4-azadodecan-12-oate (5.0 g, 15.368 mmol) obtained in Step 4 in anhydrous THF (30 mL), diisobutylaluminium hydride (DIBAL-H, 23.0 mL, 23.051 mmol) was slowly added dropwise thereto under an argon atmosphere and the mixture was stirred at 0°C for 1 hour. 10% hydrochloric acid was added to the reaction solution, stirred at 0°C for 30 minutes and the mixture was extracted with ethyl acetate. The organic layer was dried over anhydrous magnesium sulfate and filtered, and the filtrate was concentrated. After dissolving the resulting product was dissolved in methanol (18 mL) under an argon atmosphere, trimethyl orthoformate (13.4 mL, 122.941 mmol) and *p*-toluenesulfonic acid (146 mg, 0.768 mmol) were added thereto and the mixture was stirred at room temperature for 2 hours. Then, ethyl acetate was added thereto and the mixture was extracted. The organic layer was dried over anhydrous magnesium sulfate, filtered, concentrated, and purified by column chromatography. After dissolving the product again in methanol (10 mL), 10% Pd/C (76 mg, 0.4 wt%) was added thereto and the mixture was stirred at room temperature for 3 hours under hydrogen atmosphere. The reaction solution was filtered, and the filtrate was concentrated and dried under reduced pressure to give the title compound (673 mg).

¹H NMR (300 MHz, CDCl₃) δ 4.53 (t, 1H), 3.68-3.62 (m, 6H), 3.56-3.50 (m, 2H), 3.40 (s, 6H), 2.87 (t, 2H).

### Step 6. Preparation of 2-(2-(2-aminoethoxy)ethoxy)-N-(2-(2-(2,2-dimethoxyethoxy)ethoxy)ethyl)acetamide

After dissolving the 3-oxo-1-phenyl-2,7,10-trioxa-4-azadodecan-12-oic acid (952 mg, 3.204 mmol) obtained in Step 3 in acetonitrile (30 mL), (benzotriazol-1-yloxy)tris(dimethylamino)phosphonium hexafluorophosphate (BOP, 1.6 g, 3.522 mmol) and *N,N*-diisopropylethylamine (DIPEA, 1.7 mL, 9.606 mmol) were added thereto and the mixture was stirred at room temperature for 30 minutes. The 2-(2-(2,2-dimethoxyethoxy)ethoxy)ethan-1-amine (650 mg, 3.364 mmol) obtained in Step 5 was added thereto and the mixture was stirred at room temperature for 3 hours. Upon completion of the reaction, ethyl acetate was added thereto and the mixture was washed with sodium bicarbonate. Then, the resultant was dried over anhydrous magnesium sulfate and filtered, and the filtrate was concentrated and purified by column chromatography. After dissolving the product again in methanol (8 mL), 10% Pd/C (104 mg, 0.4 wt%) was added thereto and the mixture was stirred at room temperature for 3 hours under a hydrogen atmosphere. The reaction solution was filtered, and the filtrate was concentrated and dried under reduced pressure to give intermediate compound 1 (173 mg).

¹H NMR (300 MHz, MeOD) δ 4.52 (t, 1H), 4.03 (s, 2H), 3.71-3.32 (m, 22H), 2.97 (t, 2H).

### Preparation Example 2: Synthesis of intermediate (2), (S)-24-(t-butoxycarbonyl)-3-methoxy-12,21,26-trioxo-2,5,8,14,17-pentaoxa-11,20,25-triazatr itetracontan-43-oic acid, for preparation of fatty acid derivatives having at least two reactive groups

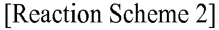

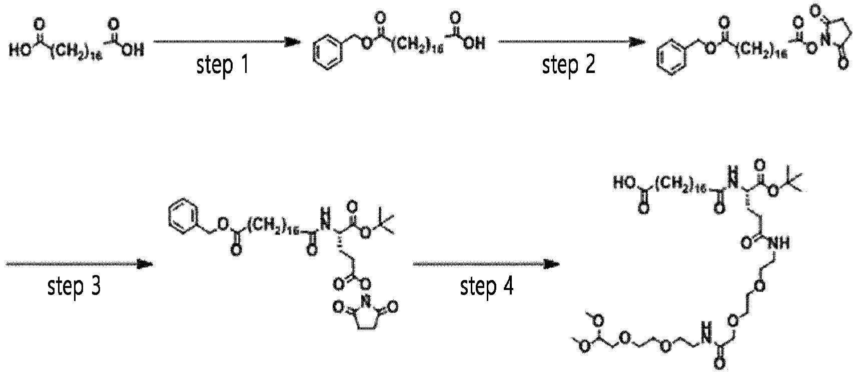

### Step 1. Preparation of 18-(benzyloxy)-18-oxooctadecanoic acid

Octadecandioic acid (100 g, 318 mmol), p-toluenesulfonic acid (756 mg, 3.975 mmol), and benzyl alcohol (26.4 mL, 254.4 mol) were added to toluene (3.7 L) and the mixture was distilled. Celite was added to the reaction solution, and this was cooled to 40°C, stirred for 1 hour, and then filtered through silica gel. The filtrate was concentrated under reduced pressure and heptane was added at 50°C. The solid was filtered off, washed with heptane, and dried to give the title compound (67.9 g).

¹H NMR (300 MHz, CDCl₃) δ 7.36-7.34 (m, 5H), 5.11 (s, 2H), 2.35 (t, 4H), 1.64 (t, 4H), 1.25 (s, 24H).

### Step 2. Preparation of 1-benzyl 18-(2,5-dioxopyrrolidin-1-yl) octadecanoate

After dissolving the 18-(benzyloxy)-18-oxooctadecanoic acid (20.0 g, 49.43 mmol) obtained in Step 1 in *N*-hydroxysuccinimide (6.8 g, 59.319 mmol) and dissolving *N,N*'-diisopropylcarbodiimide (DIC, 12.24 g, 59.312 mmol) in *N*-methyl-2-pyrrolidone (NMP, 200 mL), the mixtures were stirred at 60°C for 2.5 hours. Upon completion of the reaction, the reaction solution was cooled to room temperature and the solid was filtered off. Water was added to the filtrate and the resulting solid was filtered off and washed with water. The solid was recrystallized from isopropyl alcohol to give the title compound (21.6 g).

¹H NMR (300 MHz, CDCl₃) δ 7.37-7.31 (m, 5H), 5.11 (s, 2H), 2.83 (s, 2H), 2.60 (t, 2H), 2.35 (t, 2H), 1.77-1.53 (m, 6H), 1.25 (s, 24H).

### Step 3. Preparation of (S)-1-t-butyl 5-(2,5-dioxopyrrolidin-1-yl) 2-(18-(benzyloxy)-18-oxo-octadecanamido)pentandioate

The 1-benzyl 18-(2,5-dioxopyrrolidin-1-yl) octadecanoate (10.0 g, 19.934 mmol) obtained in Step 2 and L-glutamic acid 5-*t*-butyl ester (4.3 g, 20.931 mmol) were added to NMP (80 mL) and the mixture was stirred at 50°C for 4 hours. Upon completion of the reaction, the reaction solution was cooled to room temperature, and water (230 mL), 0.5 M potassium bisulfate (34 mL), and ethyl acetate were added thereto and the mixture was extracted. The organic layer was dried over anhydrous magnesium sulfate and filtered, and the filtrate was concentrated. After dissolving the product in NMP (75 mL), N-hydrocysuccinimide (3.7 g, 31.894 mmol) and DCC(N,n'-dicyclohexylcarbodiimide, 5.4 g, 25.914 mmol) were added thereto and the mixture was stirred at room temperature for 16 hours. The solid was filtered and the filtrate was washed with water. The organic layer was dried over anhydrous magnesium sulfate and filtered, and the filtrate was concentrated. The concentrate was purified by column chromatography to give the title compound (7.1 g).

¹H NMR (300 MHz, CDCl₃) δ 7.36-7.31 (m, 5H), 6.19 (d, 1H), 5.11 (s, 2H), 4.64-4.57 (m, 1H), 2.83 (s, 4H), 2.73-2.62 (m, 2H), 2.35 (t, 2H), 2.24-2.17 (m, 2H), 1.66-1.55 (m, 4H), 1.48 (s, 9H), 1.24 (s, 26H).

### Step 4. Preparation of (S)-24-(t-butoxycarbonyl)3-methoxy -12,21,26-trioxo-2,5,8,14,17-pentaoxa-11,20,25-triazatritetracontan-43-oic acid

The (*S*)-1-*t*-butyl 5-(2,5-dioxopyrrolidin-1-yl) 2-(18-(benzyloxy)-18-oxo-octadecanamido)pentandioate (329 mg, 0.478 mmol) obtained in Step 3, intermediate 1 prepared according to Preparation Example 1 (170 mg, 0.502 mmol), and triethylamine (0.2 mL, 1.435 mmol) were added to acetonitrile (8 mL) and the mixture was stirred at room temperature for 16 hours. Upon completion of the reaction, the reaction solution was concentrated and purified by column chromatography. After dissolving the product in methanol (10 mL), 10% Pd/C (140 mg, 0.4 wt%) was added thereto and the mixture was stirred at room temperature for 3 hours under a hydrogen atmosphere. The reaction solution was filtered, and the filtrate was concentrated and dried under reduced pressure to give intermediate compound 2 (300 mg).

¹H NMR (300 MHz, CDCl₃) δ 6.87 (br, 1H), 6.63 (d, 1H), 4.52 (t, 1H), 4.41 (m, 1H), 4.04 (s, 2H), 3.70-3.47 (m, 18H), 3.41 (s, 6H), 2.35-2.20 (m, 7H), 1.93-1.86 (m, 1H), 1.63-1.53 (m, 4H), 1.46 (s, 9H), 1.26 (s, 24H).

### Preparation Example 3: Preparation of immunoglobulin Fc fragments as biocompatible material

Immunoglobulin Fc fragments were used as a biocompatible material of a protein conjugate. The immunoglobulin Fc fragments were prepared according to the mass production method of an immunoglobulin Fc region where the initiation methionine residue is removed, disclosed in KR Pat. No. 10-0824505, which is a previous patent application by the present inventors.

### Example 1: Preparation of fatty acid derivative (1) having two reactive groups as a linker, (S)-22-(18-(2-(2,5-dioxo-2,5-dihydro-1H-pyrrol-1-yl)ethylamino)-18-oxoctadecanamido)-1,1 0,19-trioxo-3,6,12,15-tetraoxa-9,18-diazatricosan-23-oic acid

After dissolving intermediate 2 prepared according to Preparation Example 2 (150 mg, 0.183 mmol) in acetonitrile (5 mL), 1-[bis(dimethylamino)methylene]-1*H*-1,2,3-triazolo[4,5-b]pyridinium 3-oxid hexafluorophosphate (HATU, 77 mg, 0.201 mmol), DIPEA (0.1 mL, 0.549 mmol) were added thereto, and the mixture was stirred at room temperature for 20 minutes. *N*-(2-Aminoethyl)maleimide (32 mg, 0.183 mmol) was added to the reaction solution and the mixture was stirred at room temperature for 12 hours. Upon completion of the reaction, ethyl acetate was added thereto and the mixture was washed with sodium bicarbonate, and the resultant was dried over anhydrous magnesium sulfate and filtered, and the filtrate was concentrated and purified by column chromatography. After dissolving the product in dichloromethane, trifluoroacetic acid (0.16 mL, 2.123 mmol) was added thereto and the mixture was stirred at room temperature for 16 hours. The reaction solution was concentrated under reduced pressure and recrystallized with diethyl ether to give the title compound (15 mg).

¹H NMR (300 MHz, CDCl₃) δ 9.71 (s, 1H), 7.20 (br, 1H), 6.97 (br, 1H), 6.73 (s, 2H), 5.84 (br, 1H), 4.48-4.44 (m, 1H), 4.05 (s, 2H), 3.72-3.45 (m, 22H), 2.26-2.07 (m, 8H), 1.62-1.57 (m, 4H), 1.25 (s, 24H);

MS (ESI⁺): [M+H]⁺ m/z 840.5.

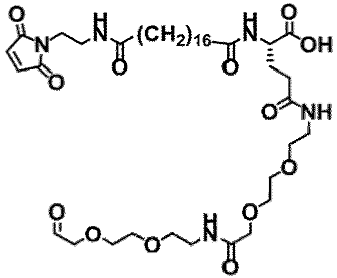

### Example 2: Preparation of fatty acid derivative (2) having two reactive groups as a linker, (S)-22-(18-(2,5-dioxopyrrolidin-1-yloxy)-18-oxooctadecanamido)-1,10,19-trioxo-3,6,12,15-te traoxa-9,18-diazatricosan-23-oic acid

After dissolving intermediate 2 prepared according to Preparation Example 2 (150 mg, 0.183 mmol) in dichloromethane (5 mL), *N*-hydroxysuccinimide (23 mg, 0.201 mmol) and 1-ethyl-3-(3-dimethylaminopropyl)carbodiimide (EDC, 42 mg, 0.219 mmol) were added thereto, and the mixture was stirred at room temperature for 16 hours. Upon completion of the reaction, the resultant was washed with sodium bicarbonate and dried over anhydrous magnesium sulfate and filtered, and the filtrate was concentrated and purified by column chromatography. After dissolving the product in dichloromethane, trifluoroacetic acid (0.17 mL, 2.181 mmol) was added thereto and the mixture was stirred at room temperature for 16 hours. The reaction solution was concentrated under reduced pressure and recrystallized with diethyl ether to give the title compound (25 mg).

¹H NMR (300 MHz, CDCl₃) δ 9.70 (s, 1H), 7.21 (d, 1H), 7.06 (br, 1H), 4.53-4.46 (m, 1H), 4.05 (s, 2H), 3.76-3.49 (m, 16H), 2.84 (s, 4H), 2.60 (t, 4H), 2.48-2.41 (m, 2H), 2.23 (t, 2H), 2.12-2.04 (m, 2H), 1.78-1.72 (m, 2H), 1.65-1.62 (m,2H), 1.25 (s, 24H);

MS (ESI⁺): [M+H]⁺ m/z 815.5.

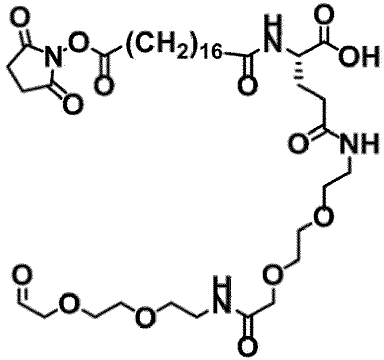

### Example 3: Preparation of conjugate in which a physiologically active polypeptide and an immunoglobulin Fc are linked through a fatty acid derivative as a linker

To link the fatty acid derivative linker containing a maleimide group and an aldehyde group as a reactive group, which were prepared according to Example 1 or 2, to a cysteine residue of a triple agonist (SEQ ID NO: 1), which is one of physiologically active polypeptide and exhibits activity to all of GLP-1, GIP, and glucagon receptors, the triple agonist and a fatty acid derivative linker were mixed at a molar ratio of 1 : 1 to 2 and reacted at 4°C to 8°C for about 1 to 2 hours. In particular, the concentration of the triple agonist was in the range of 3 mg/mL to 5 mg/mL, and the reaction was performed in the presence of 20 mM Tris (pH 7.0 to pH 8.0) and isopropyl alcohol. The triple agonist, which was linked to the fatty acid derivative linker in a molar ratio of 1:1, was purified using the buffer containing citrate (pH 3.0) and ethanol as the reaction solution and the SP-HP column (GE, U.S.A.) employing the potassium chloride concentration gradient.

Then, the purified conjugate, in which a fatty acid derivative linker and a triple agonist are linked, was reacted with an immunoglobulin Fc fragment in a molar ratio of 1 : 2 to 5 at 4°C to 8°C for 12 to 18 hours, in which the concentration of the total protein was in the range of 20 mg/mL to 35 mg/mL. In particular, the reaction solution had a pH of 6.0 to 6.5 and 20 mM sodium cyanoborohydride (SCB) was added as a reducing agent. Upon completion of the reaction, the reaction solution was applied to the Source Q column (GE, U.S.A.), which uses a bis-Tris buffer (pH 6.5) and a calcium chloride concentration gradient, and the Source ISO (GE, U.S.A.), which uses ammonium sulfate and a 20 mM Tris (pH 7.5) concentration gradient, and thereby the conjugate in which a triple agonist and an immunoglobulin Fc were linked through a fatty acid derivative linker was purified. The prepared protein conjugate was confirmed by SDS-PAGE and the results are shown in FIG. 1.

### Example 4: Preparation of protein conjugates according to kinds of fatty acids

Protein conjugates were prepared using fatty acid derivatives having 1 to 100 carbon atoms that constitute the hydrocarbon chains of the fatty acid backbones. Specifically, protein conjugates were prepared using fatty acid derivatives having 4, 5, 6, 7, 8, 9, 10, 11, 12, 13, 14, 15, 16, 17, 18, 19, 20, or 21 carbon atoms.

Specifically, protein conjugates were prepared using various kinds of fatty acid derivatives by the methods of Examples 1 to 3.

### Experimental Example 1: Confirmation of in vivo pharmacokinetics of protein conjugates

The *in vivo* pharmacokinetics of the protein conjugates prepared according to Example 4, specifically the conjugates in which triple agonists that exhibit activity to all of GLP-1, GIP, and glucagon receptors and an immunoglobulin Fc are linked through fatty acid derivatives, were confirmed, and the increase of *in vivo* duration of these protein conjugates compared to the existing triple agonists were compared. Specifically, ICR mice, which are widely used as normal animal models, were used for the confirmation of pharmacokinetics. Non-fasting 8-week-old ICR mice were divided into the following two groups after a 3-day adaptation period and administered with the test materials:
Group 1; single subcutaneous injection of triple agonists at a dose of 49.2 nmol/kg; and
Group 2; single subcutaneous injection of a conjugate, in which a triple agonist and an immunoglobulin Fc are linked through a fatty acid derivative, at a dose of 5.5 nmol/kg.

Group 1, administered with the triple agonist, consisted of 15 subjects. From these subjects, 0.3 mL of blood was cross-collected using orbital veins at 0.25, 0.5, 1, 2, and 4 hours. Meanwhile, group 2, administered with a conjugate, in which a triple agonist and an immunoglobulin Fc are linked through a fatty acid derivative, consisted of 12 subjects. From these subjects, blood samples were cross-collected at 1, 4, 8, 24, 48, 72, 96, 120, 144, and 168 hours, in the same manner as in group 1. The sera were separated from the collected samples by centrifugation (10,000 rpm, 10 minutes, Eppendorf) and stored in a -20°C freezer.

The concentrations of the triple agonists or the conjugates, in which each triple agonist and an immunoglobulin Fc are linked through a fatty acid derivative, were quantified by the enzyme-linked immunosorbent assay (ELISA) which uses triple agonist-specific antibodies. The pharmacokinetic parameters were calculated by the non-compartment method using the Phoenix™ WinNonin® 7.0 (Pharsight, U.S.A.) program based on the hourly serum concentrations of A, B, and C. The maximum blood concentration (Cₘₐₓ) and the time of arrival (Tₘₐₓ) were confirmed through the basic data, and the area under the drug concentration curve (AUC) over time was calculated by the log-linear trapezoidal summation. Other pharmacokinetic parameters such as half-life (t_{1/2}), distribution volume (V_{d}) and clearance (CL) were also calculated using the program, and the changes in blood concentration of the drug over time are shown in FIG. 2.

As shown in FIG. 2, compared to the results with regard to existing triple agonists, the conjugates according to Example 2 of the present invention, in which each triple agonist and an immunoglobulin Fc are linked through a fatty acid derivative, showed significantly increased duration of blood half-life.

These results suggest that the fatty acid linker, a novel suggestion disclosed in the present invention, which links a physiologically active polypeptide and a biocompatible material, can significantly increase the duration of the linked physiologically active polypeptide, thereby providing a status as a new drug platform capable of reducing dose and frequency of administration.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A protein conjugate, wherein a physiologically active polypeptide and a biocompatible material are linked through a fatty acid derivative.

2. The protein conjugate of claim 1, wherein the physiologically active polypeptide and the biocompatible material are each linked to the fatty acid derivative by a covalent bond.

3. The protein conjugate of claim 1, wherein the fatty acid derivative has at least two reactive groups that are linked directly or through a linker to a fatty acid backbone, and the fatty acid derivative is linked to each of the physiologically active polypeptide and the biocompatible material through the reactive groups.

4. The protein conjugate of claim 3, wherein the reactive group is 2,5-dioxopyrrolidinyl, 2,5-dioxopyrrolyl, aldehyde, aryl disulfide, heteroaryl disulfide, haloacetamide, or C₇₋₁₀ alkynyl.

5. The protein conjugate of claim 3, wherein the reactive group is maleimide, *N*-hydroxysuccinimide, succinimide, formaldehyde, acetaldehyde, propionaldehyde, butyraldehyde, orthopyridyl disulfide (OPSS), iodoacetamide, haloacetamide which comprises bromine, fluorine, chlorine, or astatin instead of iodine, difluorocyclooctyne (DIFO), dibenzocyclooctyne (DIBO), dibenzo-aza-cyclooctyne (DIBAC or DBCO), biarylazacyclooctynones (BARAC), tetramethylthiacycloheptyne (TMTH), bicyclononyne (BCN) Sondheimer diyne, cyclooctyne (OCT), monofluorinated cyclooctyne (MOFO), dimethoxyazacyclooctyne (DIMAC), 2,3,6,7-tetramethoxy-dibenzocyclooctyne (TMDIBO), sulfonylated dibenzocyclooctyne (S-DIBO), carboxymethylmonobenzocyclooctyne (COMBO), pyrrolocyclooctyne (PYRROC), or alkyne.

6. The protein conjugate of claim 3, wherein the linker is C₁₋₃ alkylamino or a (C₁₋₃ alkoxy)ₙ(C₁₋₃ alkylamino) chain (wherein n is an integer of 1 to 3).

7. The protein conjugate of claim 1, wherein at least one linked material in which the physiologically active polypeptide and the fatty acid derivative are linked is conjugated to the biocompatible material.

8. The protein conjugate of claim 1, wherein the physiologically active polypeptide is a hormone, cytokine, interleukin, interleukin-binding protein, enzyme, antibody, growth factor, transcription factor, blood factor, vaccines, structural protein, ligand protein or receptor, cell surface antigen, or receptor antagonist.

9. The protein conjugate of claim 1, wherein the physiologically active polypeptide is selected from the group consisting of:
incretins, which include glucagon-like peptide-1 (GLP-1), glucagon, gastric inhibitory polypeptides (GIPs), oxyntomodulin, xenin, insulin, cholecystokinin (CCK), amylin, gastrin, ghrelin, and peptide YY (PYY) that regulate blood glucose levels in the stomach and intestines and body weight;
adipokines, which include leptin, adiponectin, adipolin, apelin, and cartonectin that are secreted from adipose tissue;
neuropeptides, which include kisspeptin, nesfatin-1 that are secreted from the brain;
peptides or proteins, which include irisin, myonectin, decorin, follistatin, and musclin that are secreted from muscle; and
vasoactive intestinal peptides, natriuretic peptides, granulocyte colony-stimulating factor (G-CSF), human growth hormone (hGH), erythropoietin (EPO), growth hormone-releasing hormone, growth hormone-releasing peptides, interferons, interferon receptors, G protein-coupled receptors, interleukins, interleukin receptors, enzymes, interleukin-binding proteins, cytokine-binding proteins, macrophage-activating factors, macrophage peptides, B cell factors, T cell factors, protein A, allergy-inhibiting factors, necrosis glycoproteins, immunotoxins, lymphotoxins, tumor necrosis factors, tumor suppressors, metastasis growth factors, α-1 antitrypsin, albumin, α-lactalbumin, apolipoprotein-E, high-glycosylated erythropoietin, angiopoietins, hemoglobins, thrombin, thrombin receptor-activating peptides, thrombomodulin, blood factor VII, blood factor VIIa, blood factor VIII, blood factor IX, blood factor XIII, plasminogen activators, fibrin-binding peptides, urokinase, streptokinase, hirudin, protein C, C-reactive protein, renin inhibitors, collagenase inhibitors, superoxide dismutase, platelet-derived growth factor, epithelial growth factor, epidermal growth factor, angiostatin, angiotensin, bone morphogenetic growth factor, bone morphogenetic protein, calcitonin, insulin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, cholecystokinin, pancreatic polypeptides, gastrin-releasing peptides, corticotropin-releasing factor, thyroid-stimulating hormone, autotaxin, lactoferrin, myostatin, cell surface antigens, virus-derived vaccine antigens, monoclonal antibody, polyclonal antibody, and antibody fragments.

10. The protein conjugate of claim 1, wherein the physiologically active polypeptide simultaneously activates at least two receptors.

11. The protein conjugate of claim 1, wherein the biocompatible material is selected from the group consisting of polyethylene glycol (PEG), cholesterol, albumin and a fragment thereof, an albumin-binding material, a polymer of repeating units of a particular amino acid sequence, an antibody, an antibody fragment, an FcRn-binding material, *in vivo* connective tissue or a derivative thereof, a nucleotide, fibronectin, transferrin, a saccharide, a polymer, and a combination thereof.

12. The protein conjugate of claim 11, wherein the FcRn-binding material is a polypeptide which comprises an immunoglobulin Fc region.

13. The protein conjugate of claim 12, wherein the immunoglobulin Fc region is aglycosylated.

14. The protein conjugate of claim 12, wherein the immunoglobulin Fc region further comprises a hinge region.

15. The protein conjugate of claim 12, wherein the immunoglobulin Fc region is selected from the group consisting of IgG, IgA, IgD, IgE, IgM, a combination thereof, and a hybrid thereof.

16. The protein conjugate of claim 12, wherein the immunoglobulin Fc region is an IgG4 Fc fragment.

17. The protein conjugate of claim 1, wherein the fatty acid derivative is a derivative of a saturated or unsaturated fatty acid having 1 to 40 carbon atoms.

18. The protein conjugate of claim 17, wherein the fatty acid is a fatty acid selected from the group consisting of formic acid (HCOOH), acetic acid (CH₃COOH), propionic acid (C₂H₅COOH), butyric acid (C₃H₇COOH), valeric acid (C₄H₉COOH), caproic acid (C₅H₁₁COOH), enanthic acid (C₆H₁₃COOH), caprylic acid (C₇H₁₅COOH), pelargonic acid (C₈H₁₇COOH), capric acid (C₉H₁₉COOH), undecylic acid (C₁₀H₂₁COOH), lauric acid (C₁₁H₂₃COOH), tridecylic acid (C₁₂H₂₅COOH), myristic acid (C₁₃H₂₇COOH), pentadecylic acid (C₁₄H₂₉COOH), palmitic acid (C₁₅H₃₁COOH), heptadecylic acid (C₁₆H₃₃COOH), stearic acid (C₁₇H₃₅COOH), nonadecanoic acid (C₁₈H₃₇COOH), arachidic acid (C₁₉H₃₉COOH), behenic acid (C₂₁H₄₃COOH), lignoceric acid (C₂₃H₄₇COOH), cerotic acid (C₂₅H₅₁COOH), heptacosanoic acid (C₂₆H₅₃COOH), montanic acid (C₂₈H₅₇COOH), melissic acid (C₂₉H₅₉COOH), lacceric acid (C₃₁H₆₃COOH), acrylic acid (CH₂=CHCOOH), crotonic acid (CH₃CH=CHCOOH), isocrotonic acid (CH₃CH=CHCOOH), undecylenic acid (CH₂=CH(CH₂)₈COOH), oleic acid (C₁₇H₃₃COOH), elaidic acid (C₁₇H₃₃COOH), cetoleic acid (C₂₁H₄₁COOH), erucic acid (C₂₁H₄₁COOH), brassidic acid (C₂₁H₄₁COOH), sorbic acid (C₅H₇COOH, F2), linoleic acid (C₁₇H₃₁COOH, F2), linolenic acid (C₁₇H₂₉COOH, F3), arachidonic acid (C₁₉H₃₁COOH, F4), propiolic acid (CH=CCOOH), and stearolic acid (C₁₇H₃₁COOH, F1), or a derivative thereof.

19. The protein conjugate of claim 18, wherein the fatty acid is a fatty acid of palmitic acid, myristic acid, stearic acid, or oleic acid, or a derivative thereof.

20. A method for preparing a protein conjugate, comprising:
(a) linking a physiologically active peptide and a biocompatible material through a fatty acid derivative having at least two reactive groups; and
(b) separating the protein conjugate, which is a reaction product of step (a).

21. The method of claim 20, wherein step (a) comprises:
(a1) linking any one of the biocompatible material and the physiologically active polypeptide to one reactive group of the fatty acid derivative;
(a2) separating the linked material, in which any one of the biocompatible material and the physiologically active polypeptide is linked to the fatty acid, from the reaction mixture of step (a1); and
(a3) linking the other of the biocompatible material and the physiologically active polypeptide to another reactive group of the fatty acid derivative in the linked material separated in step (a2), and producing a conjugate in which the reactive groups of the fatty acid are linked to each of the physiologically active polypeptide and the biocompatible material.

22. The method of claim 21, wherein step (a1) and step (a3) are performed in the presence of a reducing agent.

23. The method of claim 22, wherein the reducing agent is sodium cyanoborohydride (NaCNBH₃), sodium borohydride, dimethylamine borane, a picoline borane complex, or borane pyridine.

24. The method of claim 21, wherein, in step (a1), the reaction molar ratio of the physiologically active polypeptide to the fatty acid derivative is in the range of 1:1 to 1:20 and the reaction molar ratio of the biocompatible material to the fatty acid is in the range of 1:1 to 1:20.

25. The method of claim 21, wherein, in step (a3), the reaction molar ratio of the linked material separated in step (a2) to the biocompatible material or the physiologically active polypeptide is in the range of 1:0.5 to 1:20.


<110>    HANMI PHARM. CO., LTD.

<120>    A protein conjugate comprising a fatty acid derivative as a
         linker and a method for preparation thereof

<130>    OPA16297-PCT

<150>    10-2015-0170929
<151>    2015-12-02

<160>    1

<170>    KoPatentIn 3.0

<210>    1
<211>    40
<212>    PRT
<213>    Artificial Sequence

<220>
<223>    Trigonal glucagon/GLP-1/GIP receptor agonist


<220>
<221>    VARIANT
<222>    (2)
<223>    Xaa = alpha-methyl-glutamic aicd or aminoisobutyric acid


<220>
<221>    VARIANT
<222>    (16)..(20)
<223>    amino acids at position 16 and position 20 form a ring


<400>    1
Tyr Xaa Gln Gly Thr Phe Thr Ser Asp Tyr Ser Lys Tyr Leu Asp Glu
  1               5                  10                  15

Lys Arg Ala Lys Glu Phe Val Gln Trp Leu Leu Asp His His Pro Ser
             20                  25                  30

Ser Gly Gln Pro Pro Pro Ser Cys
         35                  40

<110>    HANMI PHARM. CO., LTD.

<120>    A protein conjugate comprising a fatty acid derivative as a
         linker and a method for preparation thereof

<130>    OPA16297-PCT

<150>    10-2015-0170929
<151>    2015-12-02

<160>    1

<170>    KoPatentIn 3.0

<210>    1
<211>    40
<212>    PRT
<213>    Artificial Sequence

<220>
<223>    Trigonal glucagon/GLP-1/GIP receptor agonist


<220>
<221>    VARIANT
<222>    (2)
<223>    Xaa = alpha-methyl-glutamic aicd or aminoisobutyric acid


<220>
<221>    VARIANT
<222>    (16)..(20)
<223>    amino acids at position 16 and position 20 form a ring


<400>    1
Tyr Xaa Gln Gly Thr Phe Thr Ser Asp Tyr Ser Lys Tyr Leu Asp Glu
  1               5                  10                  15

Lys Arg Ala Lys Glu Phe Val Gln Trp Leu Leu Asp His His Pro Ser
             20                  25                  30

Ser Gly Gln Pro Pro Pro Ser Cys
         35                  40